# EUROPEAN PATENT SPECIFICATION

(11) **EP 2 631 232 B1**
(45) Date of publication and mention of the grant of the patent: **16.09.2015**
(21) Application number: 13150611.5
(22) Date of filing: 09.01.2013
(51) Int. Cl.: C07D 307/80, C07D 307/85, A61K 31/343, A61P 35/00

(54) **Halogen derivatives of benzo[b]furans useful as anti-neoplastic or anti-proliferative drugs**
Halogenderivate der Benzo [b] Furane und deren Verwendung als antineoplastiche und antiproliferative Arzneimittel
Dérivés halogenes de benzo [b] furanes utiles en tant que médicaments antineoplasiques et antiproliferatifs

(30) Priority: 21.02.2012 PL 39819312
(43) Date of publication of application: 28.08.2013
(73) Proprietor: Centrum Badan Molekularnych I Makromolekularnych Pan, 90-363 Lodz (PL); Warszawski Uniwersytet Medyczny, 02-091 Warszawa (PL)
(72) Inventor: Kossakowski, Jerzy, 04-854 Warszawa (PL); Kuran, Bozena, 96-320 Mszczonów (PL); Kazmierczak-Baranska, Julia, 94-123 Lódz (PL); Krolewska, Karolina, 94-003 Lódz (PL); Nawrot, Barbara, 95-100 Dabrówka Wielka (PL); Krawiecka, Mariola, 28-200 Staszow (PL); Cieslak, Marcin, 93-640 Lodz (PL)
(74) Representative: Brodowska, Iwona

(56) References cited:
- WO-A1-2004/108709
- WO-A1-2010/125350
- DATABASE CAPLUS [Online] CHEMICAL ABSTRACTS SERVICE, COLUMBUS, OHIO, US; PATEL, M. G. ET AL: "Studies on 6,7-dihydroxy-4-methylcoumarin and its methyl ethers", XP002693889, retrieved from STN Database accession no. 1960:128899 & PATEL, M. G. ET AL: "Studies on 6,7-dihydroxy-4-methylcoumarin and its methyl ethers", JOURNAL OF THE INDIAN CHEMICAL SOCIETY , 37, 227-30 CODEN: JICSAH; ISSN: 0019-4522, 1960,
- KOSSAKOWSKI J ET AL: "Synthesis and structural characterization of derivatives of 2- and 3-benzo[b]furan carboxylic acids with potential cytotoxic activity", IL FARMACO, ELSEVIER FRANCE * EDITIONS SCIENTIFIQUES ET MEDICALES, IT, vol. 60, no. 6-7, 1 June 2005 (2005-06-01) , pages 519-527, XP027697576, ISSN: 0014-827X [retrieved on 2005-06-01]

## Description

### Technical Field

The object of the invention covers the halogen derivatives of benzo[b]furans represented by formulas 1a and 1d :

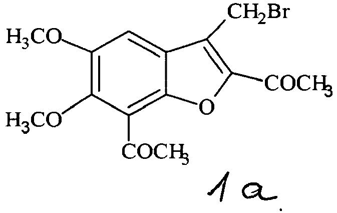

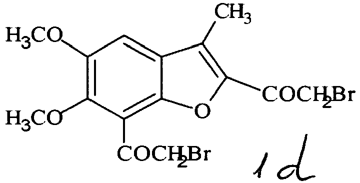

which the prior art has neither investigated nor described so far, as well as the use of the group of halogen derivatives of benzo[b]furans represented by the general formula **1** : 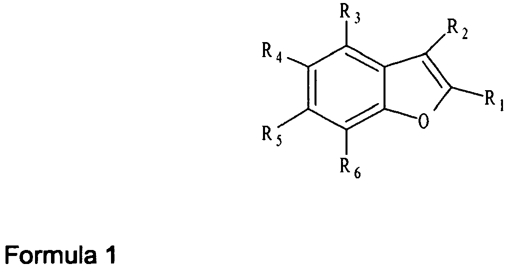 wherein:
R₁ - means independently a substituent selected from the group consisting of
-(CH₂)ₙCH₃, -COOH, -CO(CH₂)ₙCH₃, or -COO(CH₂)ₙCH₃ - type substituents, where n = 0 ÷ 6, as well as, optionally, the alkyl group is substituted by at least one halogen atom;
R₂ - means independently a substituent selected from the group consisting of
-(CH₂)ₙCH₃, -COOH, -CO(CH₂)ₙCH₃ or -COO(CH₂₎nCH₃ type substituents where n = 0 ÷ 6, as well as, optionally, the alkyl group is substituted by at least one halogen atom;
R₃ - means a -H, -OH, or -OCH₃ type substituents or a single halogen atom;
R₄ - means independently a substituent selected from the group consisting of
-OH, or -O-(CH₂)ₙCH₃ - type substituents where n = 0 ÷ 6;
R₅ - means a -OH, or -O-(CH₂)ₙCH₃ - type substituents where n = 0 ÷ 6, as well as, optionally, the alkyl group is substituted by at least one halogen atom, and
R₆ - means a -H or -CO(CH₂)ₙCH₃ type substituents where n = 0 ÷ 6, as well as, optionally, the alkyl group is substituted by at least one halogen atom, whereas
R₁, R₂, R₃, R₅, R₆ are such combinations of substituents which make the structure of the compound have at least one halogen atom, possibly in the form of pharmaceutically acceptable salts, for the purpose of production of anti-neoplastic drugs and/or anti-proliferative drugs, which have a selective impact on the neoplasms such as e.g. leukaemia, and on suspension cell cultures.

### Background Art

A number of compounds containing a benzo[b]furan system isolated from natural sources as well as synthetically obtained in the laboratory demonstrate cytotoxic and/or cytostatic activity. These are, among others, derivatives of cyclopenta[b]benzo[b]furan, arylbenzo[b]furans, benzoylobenzofurans, and derivatives of dihydrobenzofuran.

Isolated from *Aglaia* system, the derivatives of cyclopenta[b]benzo[b]furan considerably decrease the activity of a number of various cancer cells. For instance, Silvestrol demonstrates the anti-cancer activity *in vitro* and *in vivo* against the breast and prostate cancer cells [1].

Arylbenzo[b]furans display a wide range of bio-activities including:
antibacterial, cytotoxic, and anti-proliferative. They are also deemed potential immunosuppressive compounds. Among the arylbenzofurans the neoligands isolated from *Persea* show *in vitro* cytotoxic activity against cancer cells of skin and colon [2]. On the other hand, the ebenfuran III [2-(2,4-dihydroksyphenyl)-3-formyl-4-hydroksy-6-metoksy-5-(3-metyl-buten -2-yl)-benzofuran], which is isolated from *Onobrychis ebenoides,* demonstrates the activity against: bronchioloalveolar adenocarcinoma (A549) and prostatic gland adenocarcinoma (PC3) cells; renal embryonic cells transformed by adenovirus 5 (HEK-293); osteosarcoma cells (KHOS); uterine cervix cancer cells (HeLa), acute lymphoblastic leukaemia (CEM and CEM/VLB100), and large intestine adenocarcinoma (HT29) cells[3].

Hayakawa et al. proved that certain derivatives of 6-phenyl-2-arylbenzofuran would demonstrate a selective cytotoxicity against neoplastic cells VA13 [4-6].

Similarly, Xian et al. reported a preliminary toxicity data of a series of 2-arylbenzo[b]furan derivatives. Among them, 2-(3-formyl-2-(4-hydroxy-3-metoxyphenyl)-benzofuran-5-yl)-ethyl-acetals demonstrated cytotoxic activity against various cancer cell lines [7].

On the other hand, the 2-benzoyl-4-phenyl-5-hydroxybenzofuran derivatives demonstrated a high cytotoxic activity against the cells of: lymphocytic leukaemia (L1210); mammary cancer (FM3A); human lymphoblastic leukaemia (Molt/4 and CEM); uterine cervix cancer (HeLa), and myelogenous leukaemia K562 [8].

The derivatives of dihydrobenzofuran were described as inhibitors of tubulin polymerisation and as compounds showing the anti-neoplastic activity [9]. Leukaemia and breast cancer cells proved to be the most susceptible to the compounds covered by the research works.

Furthermore, the compounds containing halogens in their structures are found among the derivatives of benzofurans. In such compounds, the halogens are substituted in the benzene ring or in the benzyl substituent. The derivatives of the kind demonstrate mostly biocide, anti-arrhythmia, spasmolythic, and anti-mycotic activity [10-20]. There are few reports on anti-cancer activity of such derivatives. Examples thereof are the derivatives of 2-(3',4',5'-trimetoxybenzoyl)-benzo[b]furan which have been described as inhibitors of tubulin polymerisation [21]. They contain both electron-donor substituents, such as e.g.-CH₃, -OCH₃, -OH, and electron-acceptor substituents, such as e.g.-F, -Br, Cl, substituted in the benzene ring. Some of the said derivatives proved cytotoxic to the cancer cells of mouse lymphocytic leukaemia (L1210), mouse mammary cancer (FM3A), human lymphoblastic leukaemia (Molt/4 and CEM), and uterine cervix cancer (HeLa).

The applicant has pursued the research into benzo[b]furan derivatives showing potential pharmacological activity for many years [22-24]. Applicable publications cover certain derivatives of 2- and 3-benzo[b]furancarboxylic acids as well as benzo[b]furanol derivatives. Multidisciplinary studies into such compounds demonstrated mostly their micro-biocide and antiviral activity as well as a weak activity against the neoplasms [22-24], which makes them ineligible for the purpose of production of anti-cancer drugs. Moreover, the applicant has obtained and described certain selected derivatives of halogens [25]. Microbiological studies on such compounds demonstrated a weak antimicrobial activity.

Unexpectedly, further studies on the group of formula 1 benzo[b]furans showed that these compounds possess a high anti-neoplastic activity. This allows expecting that novel benzo[b]furan derivatives containing the halogen atoms in benzene and/or alkyl or acetyl group areas, represented by the general formula 1, possibly in the form of pharmaceutically acceptable salts, may be used for the purpose of production of anti-cancer and/or anti-proliferative drugs showing a selective activity against the neoplasms, such as e.g. leukaemiae and suspension cell cultures.

The above mentioned novel compounds were obtained by methods which the applicant had already reported in the past [25]. The structures of such compounds contain Br and Cl halogens substituted in the benzene ring and/or alkyl and acetyl groups. This location of substituents may considerably modify the anti-neoplastic activity of the reported derivatives. The novel derivatives obtained by means of the process are crystalline compounds featuring high melting points and good solubility in organic solvents.

### Summary of invention

Halogen derivatives of benzo[b]furans are represented by formulas 1a and 1d:

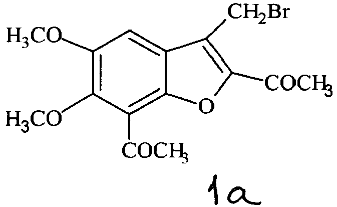

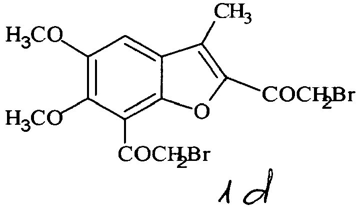

Use of halogen derivatives of benzo[b]furans represented by a general formula 1: 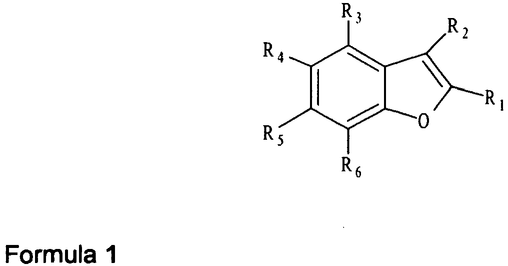 wherein:
R₁ - means independently a substituent selected from the group consisting of
-(CH₂)ₙCH₃, -COOH, -CO(CH₂)ₙCH₃, or -COO(CH₂)ₙCH₃ - type substituents, where n = 0 ÷ 6, as well as, optionally, the alkyl group is substituted by at least one halogen atom;
R₂ - means independently a substituent selected from the group consisting of
-(CH₂)ₙCH₃, -COOH, -CO(CH₂)ₙCH₃ or -COO(CH₂)ₙCH₃ type substituents where n = 0 ÷ 6, as well as, optionally, the alkyl group is substituted by at least one halogen atom;
R₃ - means a -H, -OH, or -OCH₃ type substituents or a single halogen atom;
R₄ - means independently a substituent selected from the group consisting of
-OH, or -O-(CH₂)ₙCH₃ - type substituents where n = 0 ÷ 6;
R₅ - means a -OH, or -O-(CH₂)ₙCH₃ - type substituents where n = 0 ÷ 6, as well as, optionally, the alkyl group is substituted by at least one halogen atom, and
R₆ - means a -H or -CO(CH₂)ₙCH₃ type substituents where n = 0 ÷ 6, as well as, optionally, the alkyl group is substituted by at least one halogen atom, whereas
R₁, R₂, R₃, R₅, R₆ are such combinations of substituents which make the structure of the compound have at least one halogen atom, possibly in the form of pharmaceutically acceptable salts, for the purpose of production of anti-neoplastic drugs and/or anti-proliferative drugs, which have a selective impact on the neoplasms such as e.g. leukaemia, and on suspension cell cultures.

Use of compounds described by formula 1, possibly in the form of pharmaceutically acceptable salts, for the purpose of production of anti-neoplastic drugs, specifically because of their strong toxicity against the cancer cells of chronic and acute myelogenous leukaemia, K562 and HL60, respectively.

An example of preferable new compound described by the general formula 1, as used herein, is described by formula 1a.

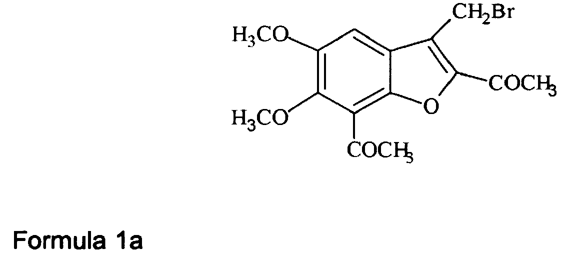

The 1a compound (1: R₁= -COCH₃, R₂=-CH₂Br, R₃=-H, R₄=-OCH₃, R₅ =-OCH₃, R₆=-COCH₃) is strongly toxic to the cancer cells of chronic myelogenous leukaemia K562 and acute myelogenous HL60. The IC50 parameter (concentration of compound producing 50% cell mortality) was determined by MTT test; after 48 hours of incubation of K562 and HL60 cells with this compound, the IC50 was **5** µM and **0.1** µM, respectively. Under the same experimental conditions, compound 1a displayed virtually zero toxicity against normal HUVEC cells (IC50 > 1 mM) and uterine cervix cancer cells HeLa (IC50 > 1 mM). For comparison, IC50 value for cis-platinum (compound used in neoplastic disease treatment) against K562 cells ranged from several to several dozens µM. Furthermore, subsequent experiments demonstrated that the incubation of compound 1a with K562 cells (1a concentration = 25 µM; incubation time = 24h) increased the activity of caspases 3 and 7, i.e. proteolytic enzymes being apoptosis markers. This indicated that the cytotoxicity of compound 1a resulted from apoptosis induction inside the cancer cells. Selective toxicity of compound 1a against the chronic and acute myelogenous leukaemia cells, absence of toxicity against normal cells, and apoptosis induction inside the cancer cells indicate that compound 1a would be useful for the production of anti-neoplastic drugs or drugs against diseases of proliferative nature.

### Conditions of 1,1'-[3-(bromomethyl)-5,6-dimethoxy-1-benzofuran-2,7-diyl] diethanon synthesis (Example I)

1,1'-(5,6-dimethoxy-3-methyl-1-benzofuran-2,7-diyl) diethanon (0.01 mole) was dissolved in CCl₄ (100 mL). Next, catalytic amounts of benzoyl peroxide (0.001 mole) and N-bromosuccynoimide (0.01 mole) were added to the reaction mixture. The mixture was refluxed for 70 h. The reaction was monitored by TLC (developing system: chloroform: methanol 9.9:0.1). When the reaction was completed, the mixture was filtered and the solvent was evaporated. Crude product was purified by column chromatography on silica gel (eluent: chloroform or chloroform/methanol 50:0.2).

### Description of embodiments

### Example I (compound 1a):

**R₁= -COCH₃, R₂= -CH₂Br, R₃=** -H, **R₄= -OCH3, R₅**= **-OCH₃, R₆= -COCH₃**

Yield: 40%; yellow powder, melting point: 104-106 °C; ¹H-NMR (300MHz, CDCl₃, δ/ppm): 2.66 (3H, s, -COCH₃), 2.73 (3H, *s*, -COCH₃), 4.01 (3H, *s*, -OCH₃), 4.25 (3H, *s*, -OCH₃), 5.33 (2H, *s*,-CH₂-Br), 7.36 (1H, *s*, Ar-H); MS (m/z): 100%= 376.9, 90%= 378.9 [L+Na⁺]

### General procedure for synthesis of bromo derivatives of benzo[b]furans (Examples II-IV)

The appropriate benzofuran (methyl 6-acetyl-5-hydroxy-2-methyl-1-benzofuran-3-carboxylate or methyl 6-acetyl-5-methoxy-2-methyl-1-benzofuran-3-carboxylate or 1,1'-(5,6-dimethoxy-3-methyl-1-benzofuran-2,7-diyl)diethanon) (0.02 mol) was dissolved in CHCl₃ (20 mL). Next, a solution of bromine Br₂ (0.04 mole) in CHCl₃ (10 mL) was added dropwise with stirring for 0.5 h. Stirring was continued for 8 h at room temperature. The reaction was controlled by TLC (developing system: chloroform: methanol 9.9:0.1). When the reaction was complete the solvent was evaporated. The residue was purified by column chromatography on silica gel (eluent: chloroform).

### Example II (compound 1b):

**R₁= -CH₃, R2= -COOCH₃, R₃= -Br, R₄= -OH, R₅= -COCHBr2, R₆= -H**

Yield: 77%; yellow crystals, melting point: 149-150 °C; ¹H-NMR (300MHz, CDCl₃, δ/ppm): 2.63 (3H, *s*, -CH₃), 3.95 (3H, s, -COOCH₃), 6.72 (1H, *s*, -COCHBr₂), 8.00 (1H, *s*, C7-H), 11.98 (1H, *s*, -OH); MS (m/z): 100%= 506.8 [L+Na⁺]
Elemental analysis for C₁₃H₉O₅Br₃:
Calculated: %C= 32.20; %H= 1.87
Found: %C= 32.38; %H= 2.09

### Example III (compound 1c):

**R₁= -CH₃, R₂= -COOCH₃, R₃=** -H, **R₄= -OCH₃, R₅=** -COCHBr2, **R₆= -H**

Yield: 78%; yellow crystals, melting point: 135-138 °C; ¹H-NMR (300MHz, CDCl₃, δ/ppm): 2.80 (3H, *s,* -CH₃), 3.98 (3H, *s,* -OCH₃), 4.05 (3H, *s,* -COOCH₃), 7.22 (1H, *s*, -COCHBr₂), 7.54 (1H, *s,* C4-H), 7.95 (1H, *s,* C7-H); MS (m/z): 100%= 443.2 [L+Na⁺]
Elemental analysis for C₁₄H₁₂O₅Br₂:
Calculated: %C= 40.03; %H= 2.88
Found: %C= 39.94; %H= 2.77

### Example IV (compound 1d):

**R₁= -COCH₂Br, R₂= -CH₃, R₃=** -H, **R₄= -OCH₃, R₅= -OCH₃, R₆= -COCH₂ Br**

Yield: 34%; yellow crystals, melting point: 148-150 °C; ¹H-NMR (300MHz, CDCl₃+ TMS, δ/ppm): 2.63 (3H, *s,* -CH₃), 4.01 (3H, *s,* -OCH₃), 4.30 *(3H, s* , -OCH₃), 4.44 (2H, *s,* -COCH₂Br), 4.46 (2H, *s,* -COCH₂Br), 7.30 (1H, *s,* Ar-H); MS (m/z): 100%= 457.1, 20%= 459.1 [L+Na⁺]

### General procedure for synthesis of chloro derivatives of benzo[b]furans (Examples V-VII)

The appropriate benzofuran (methyl 6-acetyl-5-hydroxy-2-methyl-1-benzofuran-3-carboxylate or methyl 6-acetyl-5-methoxy-2-methyl-1-benzofuran-3-carboxyliate) (0.02 mole) was dissolved in CHCl₃ (20 mL). Next chlorine, obtained in the reaction of KMnO₄ with concentrated HCl, was passed through the solution.The reaction was monitored by TLC (developing system: chloroform: methanol 9.9:0.1) the reaction was complete the solvent was evaporated. The residue was purified by column chromatography on silica gel (eluent: chloroform and chloroform: methanol 100:0.5).

### Example V (compound 1 e):

**R1= -CH3, R2= -COOCH₃, R₃= -Cl, R₄= -OH, R5= -COCHCl₂, R₆= -H**

Yield: 36%; yellow crystals, melting point: 17 1-172 °C; ¹H-NMR (300MHz, CDCl₃, δ/ppm): 2.68 (3H, *s,* -CH₃), 3.96 (3H, *s,* -COOCH₃), 6.73 (1H, *s*, -COCHCl₂), 7.98 (1H, *s*, C7-H), 11.70 (1H, *s,* -OH); MS (m/z): 100%= 350.8 [L+H⁺]
Elemental analysis for C₁₃H₉O₅Cl₃:
Calculated: %C= 44.41; %H= 2.58
Found: %C= 44.60; %H= 2.71

### Example VI

**R₁= -CH₃, R₂= -COOCH₃, R₃= -Cl, R₄=** -OH, **R₅= -COCH₂Cl, R₆=** -H

Yield: 23%; melting point: 187-188 °C; 1H-NMR δ (ppm): 12.11 (s, 1H, OH), 7.74 (s, 1H, C₇-H), 4.74 (s, 2H, COCH₂Cl), 3.96 (s, 3H, COOCH₃), 2.68 (s, 3H, CH₃); ESI MS m/z: 339.6:341 [M+Na]⁺ (100%).
Elemental analysis for C₁₃H₁₀O₅Cl₂:
Calculated: %C= 49.24; %H= 3.18
Found: %C= 49.14; %H= 3.24

### Example VII

**R₁= -CH₃, R₂= -COOCH₃, R₃=** -H, R4= **-OCH₃,** R5= **-COCH₂Cl, R₆= -H**

Yield: 54%; melting point: 130-131 °C; ¹H-NMR δ (ppm): 7.69 (s, 1H, C₇ -H), 7.19 (s, 1H, C₄-H), 4.75 (s, 2H, COCH₂Cl), 3.88 (m, 6H, COOCH₃, OCH₃), 2.60 (s, 3H, CH₃);
Elemental analysis for C₁₄H₁₃O₅Cl*2H₂O:
Calculated: %C= 50.53; %H= 3.90
Found: %C= 50.60; %H= 3.90

### Example VIII. Cytotoxic properties of halogen derivatives of benzo[b]furans

A number of halogen derivatives of benzo[b]furans were tested for cytotoxic properties against four cell lines. The tests were conducted by means of MTT where yellow tetrazole salt was processed by the dehydrogenases in mitochondria of live cells into violet formazan, in cell lines mentioned below:
- HeLa cells (uterine cervix cancer);
- HL60 cells (human acute myelogenous leukaemia);
- K562 cells (human chronic myelogenous leukaemia), and
- HUVEC cells (normal human endothelial cells).
   1. HeLa, K562, HL 60, and HUVEC cells, 7 thousand cells per well (in 200 µl of the medium) were disseminated on 96-well plates. Cells were incubated overnight in the incubator (37°C, 5% CO₂).
   2. Test compounds were dissolved in DMSO; 2µL solution amounts were added to the cells. Final concentrations of the culture: 1mM, 10µM, 100nM, 1nM. Control: DMSO-containing cells, final concentration of which amounted to 1% in the culture.
   3. Cytotoxicity test (MTT) of test compounds after 48h incubation. After 48h incubation of HeLa, K562, HL60, or HUVEC cells with test compounds, 25 µl of MTT per well added (input concentration = 5mg/ml) to the mixture. MTT, yellow tetrazole salt was reduced to violet formazan by mitochondrial dehydrogenases of live cells; the volume of formazan was measured spectrophotometrically. After 2h incubation of cells with MTT in the incubator (37°C; 5% CO₂), cells were lysed in lysing buffer containing SDS and DMF (95 µl/well). The lysis was conducted overnight at 37°C and with 5% CO₂.
   4. MTT results were taken from FLUOstar Omega reader (wavelengths: 570 and 650 nm). Absorbance measured at 570 nm was proportional to the number of live cells. The plates were analysed by reading out the absorbance values at 570 nm and 650 nm wavelengths. The values of IC50 (concentration of compound producing a 50% cell mortality) was taken from the diagram by means of interpolation for a 50% cell survival rate.

The results (shown in the table below) indicate a high and selective toxicity of compound **1a** against the K562 and HL60 cells of chronic and acute myelogenous leukaemia.

**Table 2**

| Compound | HeLa | K562 | HL60 | HUVEC |
|---|---|---|---|---|
| | IC50 48h | IC50 48h | IC50 48h | IC50 48h |
| 1a | > 1 mM | **5 µM** | **0.1 µM** | > 1 mM |
| 1b | **9 µM** | **1 µM** | **4 µM** | > 1 mM |
| 1c | **1 µM** | **1 µM** | **4 µM** | > 1 mM |
| 1e | **10 µM** | > 1 mM | **3 µM** | > 1 mM |

Strong toxicity against the K562 and HL60 cells of chronic and acute myelogenous leukaemia was detected after 48 hours of incubation with compound 1a. Test compound was toxic to neither uterine cervix cancer cells (HeLa) nor normal HUVEC cells (umbilical vein epithelium). The above mentioned results imply that compound 1a is **selectively** toxic to the neoplasms of leukaemia type and **is not toxic to normal cells.** Compounds 1b and 1c are toxic to HeLa, K562, and HL60 cells. In contrast, they are not toxic to normal cells (HUVEC). On the other hand, compound 1e is specifically toxic to uterine cervix cancer cells (HeLa) and acute promyelocytic leukaemia cells (HL60); contrary to the above, it is not toxic to chronic myelogenous leukaemia cells (K562) and normal cells.

### Example IX: Toxicity mechanism of halogen-derivative compounds of benzo[b]furans.

The above mentioned toxicity of compound 1a was tested for its origin, i.e. induction of necrosis or apoptosis in the K562 cells of chronic myelogenous leukaemia. Accordingly:
- K562 cells in RPMI 1640 medium containing a 10% calf foetal serum and antibiotics, were disseminated on a 96-well plate, in the amount of 20 x 10³ cells per well. Cells were cultured for 24 hours in the incubator (5% CO₂ ; 37°C).
- Compound 1a was dissolved in DMSO and added to the cell medium; final concentration in the cell culture was 25 µM (5 x IC50). Negative control was established by means of cells cultured in the presence of DMSO at the concentration of 1%. Positive control was established by means of cells cultured in the presence of 1 µM staurosporin, a strong apoptosis inductor. The cells were incubated with the compounds for 18 hours in the incubator (5% CO₂; 37°C).
- The test to determine the activity of captase 3 and 7 was conducted by means of Apo-ONE^{®} Homogeneous Caspase-3/7 Assay test set (Promega, Madison, Wl, USA), in compliance with manufacturer's guidelines. Cells were lysed and incubated with a pro-fluorescent substrate for caspases for 1.5 hours at room temperature. Due to proteolytic activity of caspases, the pro-fluorescent substrate was transformed into a fluorescent product the amount of which could be determined by means of fluorescence intensity measurement.
- Fluorescence intensity in each well was measured on the plate by means of FLUOStar Omega (BMG-Labtech, Germany) plate reader, at the exciting light wavelength of 485 nm and emission of 520 nm. Fluorescence intensity was proportional to the activity of caspase 3 and 7 in the sample.

### RESULTS

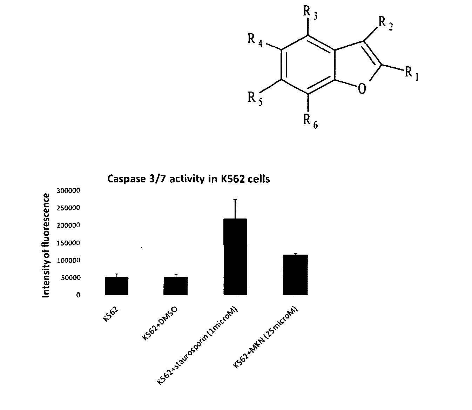

Compared with reference cells (K562 and K562+DMSO), the activity of caspase 3 and 7 was found (Fig. 1) higher in chronic myelogenous leukaemia K562 cells incubated with compound 1a at the concentration of 25 µM. This indicates that the cytotoxicity of compound 1a results from the induction of apoptosis in the cells of chronic myelogenous leukaemia (K562).

### Citation list

1. Cencic, Regina: Carrier, Marilyn; Galicia-Vázquez, Gabriela; Bordeleau, Marie-Eve; Sukarieh, Rami; Bourdeau, Annie; Brem, Brigitte; Teodoro, José G.; Greger, Harald; Tremblay, Michel L.; Porco, Jr., John A.; Pelletier, Jerry: Antitumor Activity and Mechanism of Action of the Cyclopenta[b]benzofuran, Silvestrol; PLoS ONE, April 2009, Vol. 4, Issue 4, e5223.
2. Tsai, Ian-Lih; Hsieh, Chih-Feng, and Duht, Chang-Yih: Additional Cytotoxic Neolignans From Persea Obvatifolia; Phytochemistry, 1998, Vol. 48, No. 8, pp. 1371-1375.
3. Katsanou, Efrosini S.; Halabalaki, Maria; Aligiannis, Nektarios: Mitakou, Sofia; Skaltsounis, Alexios-Leandros; Alexi, Xanthippi; Pratsinis, Harris; Alexis, Michael N.: Cytotoxic effects of 2-arylbenzofuran phytoestrogens on human cancer cells: Modulation by adrenal and gonadal steroids; Journal of Steroid Biochemistry & Molecular Biology, 104, 2007, 228-236.
4. Hayakawa, I.; Shiota, R.; Agatsuma, T.; Furukawa, H.; Naruto, S.; Sugano, Y.: Bioorg Med Chem Lett 2004; 14:455. [PubMed: 14698180].
5. Hayakawa, I.; Shiota, R.; Agatsuma, T.; Furukawa, H.; Naruto, S.; Sugano, Y.: Bioorg Med Chem Lett 2004; 14:4383. [PubMed: 15357958].
6. Hayakawa, I.; Shiota, R.; Agatsuma, T.; Furukawa, H.; Naruto, S.; Sugano, Y.: Bioorg Med Chem Lett 2004; 14:3411. [PubMed: 15177443].
7. Yang, H.; Pang, J.Y.; Cai, Y.C.; Xu, Z.L.; Xian, L.J.J.: Pharm Pharmacol 2006; 58:1281.
8. Romagnoli, Romeo; Baraldi, Pier Giovanni; Sarkar, Taradas; Lopez Cara, Carlota; Cruz Lopez, Olga; Carrion, Maria Dora; Preti, Delia; Tolomeo, Manlio; Balzarini, Jan and Hamel, Ernest: Synthesis and Biological Evaluation of 2-aroyl-4-phenyl-5-hydroxybenzofurans as a New Class of Antitubulin Agents; Med Chem. 2008 November ; 4(6): 558-564.
9. Pieters, Luc; Van Dyck, Stefaan; Gao, Mei; Bai, Ruoli; Hamel, Ernest; Vlietinck, Arnold and Lemière, Guy: Synthesis and Biological Evaluation of Dihydrobenzofuran Lignans and Related Compounds as Potential Antitumor Agents that Inhibit Tubulin Polymerisation; J. Med. Chem., 1999, 42 (26), pp. 5475-5481.
10. Kodama, I.; Kamiya, K.; Toyama; Amiodarone, J.: Ionic and cellular mechanisms of action of the most promising class III agent. Am. J. Cardiol. 1999, 84, 20R-28R.
11. Courchesne, W.E.: Characterization of novel, broad-based fungicidal activity for anti-arrhythmic drug amiodarone. J. Pharmacol. Exp. Ther. 2002, 300, 195-199.
12. Courchesne, W.E.; Ozturk, S.: Amiodarone induces a caffeine-inhibited, MID1-dependent rise in free cytoplasic calcium in Saccharomyces cerevisiae. Mol. Microbiol. 2003, 47, 223-234.
13. Nattel, S.; Singh, B.N.: Evolution, mechanisms, and classifications of anti-arrhythmic drugs: Focus on class III actions. Am. J. Cardiol. 1999, 84, 11R-19R.
14. Gill, J.; Heel, R.C.; Fitton, A.: Amiodarone. An overview of its pharmacological properties, and review of its therapeutic use in cardiac arrhythmias. Drugs 1992, 43, 69-110.
15. Pizzichini, M.; Aleo, M.F.; Marcolongo, R.; Marinello, E.: The mechanism of benziodarone activity. Quad Sclavo Diagn 1982, 18, 203-208.
16. Heel, R.C.; Brogden, R.N.; Speight, T.M.; Avery, G.S.: Benzbromarone: A review of its pharmacological properties and therapeutic use in gout and hyperuricaemia. Drugs 1977, 14, 349-366.
17. Masbernard, A.; Giudicelli, C.P.: Ten years' experience with benzbromarone in the management of gout and hyperuricaemia. S. Afr. Med J. 1981, 59, 701-706.
18. Repolles, M.J.; Pubill, C.F.; Cabeza, L.L.; Carbo, B.M.; Cerda, R.J.A.; Negrie, R.C.: Benzofuran, dihydrobenzofuran, dihydrobenzopyran and benzopyran derivatives as antidepressant agents. *Spanish Patent* ES2131020 A1, 1999.
19. Graves, A.P.; Brenk, R.; Shoichet, B.K.: Decoys for docking. J. Med. Chem. 2005, 48, 3714-3728.
20. Matsuda, S.; Ishii, M.; Kitajiri, N.; Yahara, I.; Harada, H.; Yonetani, Y.: Antihypertensive property of a novel uricosuric diuretic, S-8666, in rats. Drug Dev. Res. 1989, 17, 207-217.
21. Romagnoli, Romeo; Baraldi, Pier Giovanni; Carrion, Maria Dora; Lopez Cara, Carlota; Cruz Lopez, Olga; Tolomeo, Manlio; Grimaudo, Stefania; Di Cristina, Antonietta; Pipitone, Maria Rosaria; Balzarini, Jan; Zonta, Nicola; Brancale, Andrea; Hamel, Ernest: Design, synthesis and structure-activity relationship of 2-(3',4',5'-trimethoxybenzoyl)-benzo[b]furan derivatives as a novel class of inhibitors of tubulin polymerisation. Bioorganic & Medicinal Chemistry 17 2009, 6862-6871.
22. Kossakowski, J.; Ostrowska, K.; Hejchman, E.; Wolska, I.: Synthesis and structural characterisation of derivatives of 2- and 3-benzo[b]furan carboxylic acids with potential cytotoxic activity. II Farmaco 2005, 60, 519-527.
23. Kossakowski, J.; Ostrowska, K.: Synthesis of new derivatives of 2,3-dihydro-7-benzo[b]furanol with potential pharmacological activity. Acta Pol. Pharm. 2006, 63, 271-275.
24. Kossakowski, J.; Ostrowska, K.; Struga M.; Stefańska, J.: Synthesis of new derivatives of 2,2-dimethyl-2,3-dihydro-7-benzo[b]furanol with potential antimicrobial activity. Med. Chem. Res. 2009, 18, 555-565.
25. Kossakowski, Jerzy; Krawiecka, Mariola; Kuran, Bozena; Stefańska, Joanna and Wolska, Irena: Synthesis and Preliminary Evaluation of the Antimicrobial Activity of Selected 3-Benzofurancarboxylic Acid Derivatives; Molecules 2010, 15, 4737-4749; doi:10.3390/molecules15074737.

## Claims

1. Halogen derivatives of benzo[b]furans represented by formulas 1a and 1d :

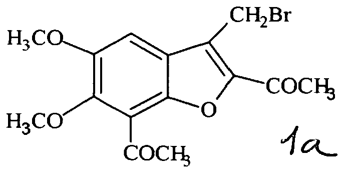

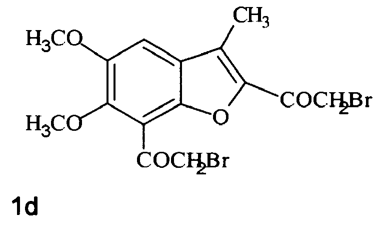

2. Use of halogen derivatives of benzo[b]furans represented by general formula **1** 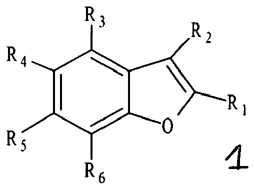 wherein: R₁ - means independently a substituent selected from the group consisting of -(CH₂)ₙCH₃, -COOH, -CO(CH₂)ₙCH₃, or -COO(CH₂)ₙCH ₃ - type substituents, where n = 0 ÷ 6, as well as, optionally, the alkyl group is substituted by at least one halogen atom; R₂ - means independently a substituent selected from the group consisting of -(CH₂)ₙCH₃, -COOH, -CO(CH₂)ₙCH₃ or -COO(CH₂)ₙCH₃ type substituents where n = 0 ÷ 6, as well as, optionally, the alkyl group is substituted by at least one halogen atom; R₃ - means a -H, -OH, or -OCH₃ type substituents or a single halogen atom; R₄ - means independently a substituent selected from the group consisting of -OH, or -O-(CH₂)ₙCH₃ - type substituents where n = 0 ÷ 6; R₅ - means a -OH, or -O-(CH₂)ₙCH₃ - type substituents where n = 0 ÷ 6, as well as, optionally, the alkyl group is substituted by at least one halogen atom, and R₆ - means a -H or -CO(CH₂)ₙCH₃ type substituents where n = 0 ÷ 6, as well as, optionally, the alkyl group is substituted by at least one halogen atom, whereas R₁, R₂, R₃, R₅, R₆ are such combinations of substituents which make the structure of the compound have at least one halogen atom, possibly in the form of pharmaceutically acceptable salts, for the purpose of production of anti-neoplastic drugs and/or anti-proliferative drugs, which have a selective impact on the neoplasms such as e.g. leukaemia, and on suspension cell cultures.

3. Use according to claim 2, for the purpose of production of drugs applied in anti-proliferative type treatments against normal and cancer cells, including chronic and acute myelogenous leukaemias and uterine cervix carcinomas, separately or combined with the existing drugs.

## Patentansprüche

1. Halogen-Derivate von Benzo[b]Furane durch die Formeln 1a und 1d dargestellt wird:

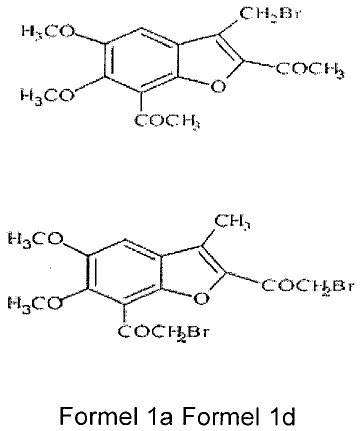

2. Verwenden Halogenderivate des Benzo[b]Furane durch die allgemeine Formel 1 vertreten 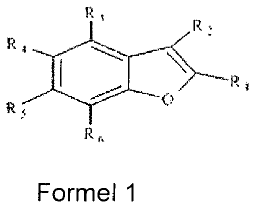 R₁ - bedeutet, unabhängig einen Substituenten, ausgewählt aus der Gruppe bestehend aus -(CH₂)ₙCH₃, -COOH, -CO(CH₂)ₙCH₃ oder -COO(CH₂)ₙCH₃ - Typ Substituenten, wobei n = 0 ÷ 6, sowie gegebenenfalls die Alkylgruppe durch mindestens ein Halogenatom substituiert ist; R₂ - bedeutet, unabhängig voneinander einen Substituenten ausgewählt aus der Gruppe bestehend aus -(CH₂)ₙCH₃, -COOH, -CO(CH₂)ₙCH₃ oder -COO(CH₂)ₙCH₃ Typ Substituenten, wobei n = 0 ÷ 6, sowie gegebenenfalls das Alkylgruppe durch mindestens ein Halogenatom substituiert ist; R₃ - bedeutet ein -H, -OH oder -OCH₃ Typ Substituenten oder eine einzige Halogenatom ist; R₄ - bedeutet unabhängig voneinander einen Substituenten, ausgewählt aus der Gruppe bestehend aus -OH oder -O-(CH₂)ₙCH₃ - Typ Substituenten, wobei n = 0 ÷ 6; R₅ - bedeutet eine -OH oder -O-(CH₂)ₙCH₃ - Typ Substituenten, wobei n = 0 ÷ 6 sowie gegebenenfalls ist die Alkylgruppe durch mindestens ein Halogenatom und R₆ substituierten - bedeutet ein -H oder -CO(CH₂)ₙCH₃ Typ Substituenten, wobei n = 0 ÷ 6 sowie gegebenenfalls ist die Alkylgruppe durch mindestens ein Halogenatom substituiert ist, während R₁, R₂, R₃, R₄, R₅, R₆ sind solche Kombinationen Als Substituenten machen die Struktur der Verbindung mindestens ein Halogenatom substituiert, gegebenenfalls in Form von pharmazeutisch verträglichen Salzen, zum Zwecke der Produktion von antineoplastischen Arzneimittel und / oder anti-proliferative Wirkstoffe, die eine selektive Wirkung auf die aufweisen Neoplasmen wie zB Leukämie, und über die Aussetzung Zellkulturen.

3. Verwendung nach Anspruch 2, zum Zwecke der Herstellung von Arzneimitteln in anti-proliferative Aktivität Behandlungen gegen normale und Krebszellen, einschließlich chronischer und akuter myeloischer Leukämie und Gebärmutterhalskarzinomen aufgebracht, getrennt oder zusammen mit den existierenden Medikamenten kombiniert.

## Revendications

1. dérivés halogènes de benzo[b]furanes représentés par des formules 1a et 1d:

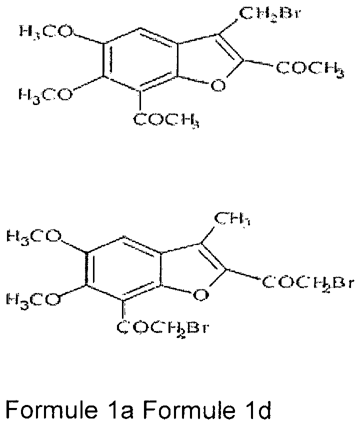

2. Utilisez dérivés halogénés de benzo[b]furannes représenté par une formule générale 1 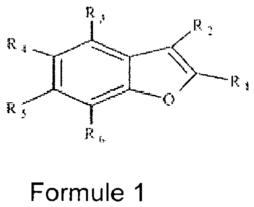 R₁ - signifie indépendamment un substituant choisi dans le groupe constitué par -(CH₂)ₙCH₃, -COOH, -CO(CH₂)ₙCH₃, ou -COO(CH₂)ₙCH₃ - substituants de type, où n = 0 ÷ 6, ainsi que, éventuellement, le groupe alkyle est substitué par au moins un atome d'halogène; R₂ - signifie indépendamment un substituant choisi dans le groupe constitué par -(CH₂)ₙCH₃, -COOH, - CO(CH₂)ₙCH₃ ou -COO substituants de type (CH₂)ₙCH₃ où n = 0 ÷ 6, ainsi que, éventuellement, la groupe alkyle est substitué par au moins un atome d'halogène; R₃ - un moyen de -H, -OH, -OCH₃ ou substituants de type ou un seul atome d'halogène; R₄ - désigne indépendamment un substituant choisi dans le groupe constitué par -OH, ou -O-(CH₂)ₙCH₃ - substituants où n = 0 ÷ 6 Type; R₅ - désigne un groupe -OH, ou -O-(CH₂)ₙCH₃ - substituants du type où n = 0 ÷ 6, ainsi que, éventuellement, le groupe alkyle est substitué par au moins un atome d'halogène, R₆ et - un moyen de -H ou -CO(CH₂)ₙCH₃ substituants du type où n = 0 ÷ 6, ainsi que, éventuellement, le groupe alkyle est substitué par au moins un atome d'halogène, alors R₁, R₂, R₃, R₄, R₅, R₆ sont telles combinaisons des substituants qui rendent la structure du composé possède au moins un atome d'halogène, éventuellement sous forme de sels pharmaceutiquement acceptables, aux fins de la production de médicaments anti-néoplasiques et / ou des médicaments anti-prolifératifs, qui ont un effet sélectif sur la néoplasmes tels que par exemple la leucémie, et sur des cultures cellulaires en suspension.

3. Utilisation selon la revendication 2, en vue de la production de médicaments appliqués dans les traitements de type anti-prolifératifs contre les cellules normales et cancéreuses, y compris les leucémies et chronique myélogène aiguë et des carcinomes du col utérin, séparément ou en combinaison avec les médicaments existants.
